# EUROPEAN PATENT APPLICATION

(11) **EP 4 269 564 A1**
(43) Date of publication of application: **01.11.2023**
(21) Application number: 20966596.7
(22) Date of filing: 25.12.2020
(51) Int. Cl.: C12N 5/071, C12N 5/0793, A61K 35/30, A61P 17/02

(54) **CHEMICAL INDUCTION METHOD FOR PHOTORECEPTOR NEURON CELLS**

(71) Applicant: Iregene Therapeutics Ltd, Wuhan, Hubei 430074 (CN)
(72) Inventor: WEI, Jun, Wuhan, Hubei 430074 (CN); CAI, Meng, Wuhan, Hubei 430074 (CN); NIU, Lumeng, Wuhan, Hubei 430074 (CN)
(74) Representative: Altmann Stößel Dick Patentanwälte PartG mbB
(86) International application number: PCT/CN2020/139521
(87) International publication number: WO 2022/134031

(57) **Abstract**

Provided is a pure chemical induction method for photoreceptor neuron cells. A group of small molecule inhibitors are added into a serum-free basic culture medium, and pluripotent stem cells are quickly converted into photoreceptor neuron cells. In the present invention, serum is not used, and chemical small molecules are used to replace retinol, eliminating the adverse factors of serum while ensuring the operation of visual circulation required by the development of photoreceptor neurons.

## Description

### FIELD OF THE INVENTION

The present invention belongs to biological field. It specifically relates to a chemical induction and culture method of photoreceptor neuron cells, the culture medium and the use thereof in various diseases.

### BACKGROUND OF THE INVENTION

The ectoderm is the outermost layer formed during embryonic development. With the beginning of organogenesis, ectoderm cells gradually differentiate into important systems such as brain, spinal cord, and sensory organs. The nervous system is an important system responsible for thinking, emotion, perception, movement and other functions. Compared with diseases such as tumors, there are fewer drugs for neurological diseases, and the development cycle thereof is long. One of the most important reasons is the specialityof various primary cells in the ectodermal lineage, for example, the unreproducibility of primary neurons results in the scarcity of in vitro drug screening platforms for nervous system drugs. In vitro regeneration of ectoderm cells can solve a variety of degenerative diseases. For example, neurodegenerative diseases are common aging diseases, wherein the cost of treatment and care is extremely expensive, and there is no specific drug on the market for effective treatment. Neurodegenerative diseases include amyotrophic lateral sclerosis (ALS), Parkinson's disease (PD), Alzheimer's disease (AD) and other diseases. According to the statistics of the World Health Organization, there will be more than 30 million patients with neurodegenerative diseases in China in 2050, and the expected medical expenses will be more than 1 trillion. The difficulty in the treatment of such diseases is that the nerve cells of central nervous system are non-renewable, and these diseases are caused by irreversible damage to the central nervous system. At present, the transplantation of nerve cells is the most effective treatment method.

Degenerative diseases in optic nerve also belong to the neurodegenerative diseases. Taking the age-related macular degeneration (AMD) as an example, it is a leading cause of vision impairment in people over 50 years old, characterized by degeneration of retina, which can lead to loss of central vision. In addition, pigmental degeneration, a rare genetic disease, is characterized by the gradual degeneration of photoreceptors in the retina, which cannot convert light signals into electrical signals to be transmitted to the brain. The retina is an important tissue that can perceive "images" in higher animals and is composed of photosensitive cells and photoreceptors. Photoreceptors can convert the light signals of an image into neural signals, and are important cells that allow the brain to "see" the image. Wherein, cone receptor cells (i.e., cone cells), are responsible for our daytime vision and color perception; rod cells are more sensitive, and can perceive weak light even in low light conditions. Damage to photoreceptor cells is an important cause of retinal degenerative diseases, resulting in the inability of eyeball to transmit the nerve signals converted from "images" to the brain, which seriously affects the vision of the subject and even leads to blindness. What's more serious is that the damage of photoreceptor nerve cells is an irreversible process and cannot be repaired by itself, so there is no good treatment method for such diseases. Retinal transplantation is used in some patients, but retinal resources are limited, mostly from cadaver donations, which are far from meeting clinical needs. The principle of artificial retina is to replace these photoreceptors. The artificial retina consists of implants anchored under the retina and electrode compounds that stimulate retinal neurons to transmit information to the brain, but current artificial retinas are expensive and do not restore visual function delicately. There is also a gene therapy based on viral vectors, which restores the function of hereditary photoreceptors by correcting genetic mutations. Similarly, the gene therapy, in addition to being expensive, can not cure the degenerative diseases in optic nerve caused by age.

In 2006, Shinya Yamanaka's team invented a "cocktail" method composed of four transcription factors (i.e., OCT4, SOX2, KLF4 and c-Myc), which can successfully reprogram terminally differentiated skin fibroblasts into stem cells with differentiated pluripotency. Such stem cells are called induced pluripotent stem cells (Cell, 2006, 124(4) pp.663-676; Cell, 2007, 131(5) pp.861-872). These stem cells have a similar differentiation potential to embryonic stem cells, and can form the three most basic germ layers in human development: ectoderm, mesoderm and endoderm, and can eventually form a variety of somatic cells. This invention breaks through the ethical limitation of using human embryonic stem cells in medicine, can solve the problem of immune rejection in cell transplantation therapy, and greatly expands the application potential of stem cell technology in clinical medicine. The induction or differentiation of ectoderm cells by using totipotential stem cells or pluripotent stem cells (including embryonic stem cells and iPSCs) as raw materials can be used as a new idea for clinical therapy, greatly expanding the application potential of ectoderm cells in clinical medicine.

Taking the nervous system as an example, currently in the field of regeneration medicine, the induction of neural stem cells and neurons mostly adopts a method of Dual SMAD inhibition (Nat Biotechnol, 2009, 05; 27(5): 485), wherein the neural stem cells obtained by this method can differentiate into other types of neuron cells. The principle of the method is to simulate the signaling pathway in early embryonic development by inhibiting BMP and TGFBeta pathways, thereby inducing the generation of neural stem cells. Among them, LDN-193189 and SB431542 are two widely used chemical small molecule inhibitors, which respectively act on ALK2 and ALK3 in the BMP4 pathway and ALK5 in the TGFBeta pathway to achieve the formation of transplanted endoderm and mesoderm, thereby playing a role of neurogenesis. Through this method, photoreceptor neurons can be obtained in the presence of serum components (Knockout Serum Replacement) fetal bovine serum (FBS) and retinoic acid, with a production cycle of at least 70 days (STEM CELLS TRANSLATIONAL MEDICINE, 2018; 7:210-219; Sci Transl Med, 2019.1 16; 11(475)). However, the presence of serum extracts and animal-derived extracts is a potential threat for drug safety in the field of cell therapy, especially cell medicament; for example, the incorporation of animal-derived viruses and the presence of allergenic proteins may lead to failure of the therapeutic effect. Furthermore, retinoic acid is an important component in the visual cycle, and is involved in the following processes: conversion of vitamin A (known as all-trans retinol or all-trans ROL) into 11-cis retinal ( 11-cis-RAL) by Retinal Pigment Epithelium (RPE), transportation of 11-cis-RAL and conversion into photoreceptors, generation of photopigments, isomerization and reduction of 11-cis-RAL to all-trans ROL, and transportation of all-trans ROL back to RPE for participating in another cycle (Nature. 1960; 168: 114-118; Nat. Genet. 1997; 15: 236-246; J. Biol. Chem. 1997; 272: 10303-10310). Retinoids in cell culture systems, such as retinoic acid (RA), are highly susceptible to oxidation and inactivation. Therefore, in order to stabilize retinoic acid from rapid oxidative damage, serum is widely used to stabilize retinoic acid in cell culture systems (Int J Dev Biol. 2012;56(4):273-8). Therefore, a retinoid chemical molecule that is both serum-independent and stable under cell culture conditions is extremely important for the stable and safe induction of photoreceptor neurons in vitro.

Galunisertib (LY2157299) used in the present invention, named as 4-(2-(6-methylpyridin-2-yl)-5,6-dihydro-4H-pyrrolo[1,2-b]pyrazol-3-yl) quinoline-6-carboxamide or 4-(2-(6-methylpyridin-2-yl)-5,6-dihydro-4H-pyrrolo[1,2-b]pyrazol-3-yl)quinoline-6-carboxamide, is a small molecule inhibitor for TGF-β receptor I (TGF-βRI, ALK5). LY2157299 is currently in phase II clinical evaluation for its anticancer activity against liver cancer and glioblastoma (Giannelli G1, Villa E, Lahn M. Transforming Growth Factor-β as a Therapeutic Target in Hepatocellular Carcinoma. Cancer Res. 2014.4.1; 74(7):1890-4), which inhibits tumor growth, invasion and metastasis by blocking the TGF-β signaling pathway. In addition, multiple studies have shown that the LY2157299 blocks the production of CTGF and inhibits neovascularization, thereby inhibiting the growth of cancer cells. At present, researches on this molecule are focused on drug development and treatment of lung cancer, liver cancer and glioblastoma (Pharmaceutics. 2020.5.18; 12(5):459), and its application in the field of nerve regeneration has not disclosed.

The present invention provides a serum-free small molecule chemical induction system, wherein pluripotent stem cells are rapidly converted into photoreceptor nerve cells. Compared with the current induction method of serum plus retinol, in the present invention, serum is not used, and chemical small molecules are used to replace retinol, eliminating the adverse factors of serum while ensuring the operation of visual circulation required by the development of photoreceptor neurons. Therefore, the present invention not only completely avoids the potential threat introduced by animal-derived components during the cell culture process, but also exhibits great advantages in purity and yield through the novel combination of chemical small molecules. Therefore, the present invention greatly expands the clinical prospect of photoreceptor nerve cell transplantation. At the same time, the present invention also solves the problems of low purity and long cycle in the production process of similar cell products, so it has huge economic and social effects.

### SUMMARY OF THE INVENTION

The present invention relates to a chemical induction and culture method of photoreceptor neuron cells, the culture medium and the use thereof in various diseases.

Specifically, the present disclosure relates to the following aspects:
In one aspect, the present invention provides use of LY2157299 in the differentiation of (for example, human-derived) pluripotent stem cells into ectoderm.

In another aspect, the invention provides a medium for differentiation from (for example, human-derived) pluripotent stem cells into (for example, human-derived) ectoderm.

Furthermore, the culture medium for differentiating (for example, human-derived) pluripotent stem cells into (for example, human-derived) ectoderm is characterized in that LY2157299 is added to a NouvNeu-basal base medium; wherein the NouvNeu-basal base medium comprises Duchenne's modified Eagle/F12 medium (DMEM/F12 medium), 0.1%-2% Minimum Essential Medium Non-Essential Amino Acids (the optimal concentration is 1%), 0.1-0.8 g/L sodium chloride (the optimal concentration is 0.5g/L), 0.1-80mg/L vitamin C (L-ascorbic acid, the optimal concentration is 64mg/L), 0.1-1.8uM vitamin B12 (the optimal concentration is 1.2uM), 0.1-9.8ng/ml progesterone (the optimal concentration is 6.3ng/ml), 10-35ug/ml putrescine (the optimal concentration is 23ug/ml), 1ug /L-25ug/L sodium selenite (the optimal concentration is 13.6ug/L), 0.1-44ug/ml insulin (the optimal concentration is 22ug/ml), 0.5-20umol/L Optiferrin (the optimal concentration is 10µmol/ L), 2.5-100ng/ml plant-derived recombinant human basic growth factor (OsrbFGF, the optimal concentration is 50ng/mL).

Further, the amount of LY2157299 is 55nM-25uM, preferably 100nM, 300nM, 500nM, 700nM, 900nM, 1uM, 2.5uM, 5uM, 7.5uM, 10uM, 12.5uM, 15uM, 17.5uM, 20uM or 22uM.

Further, the amount of LY2157299 is 7.5uM.

In another aspect, the present invention provides a method for differentiating (for example, human-derived) pluripotent stem cells into (for example, human-derived) ectoderm, wherein the pluripotent stem cells are cultured in a NouvNeu-basal base medium supplemented with 55nM-25uM LY2157299 (preferably 100nM, 300nM, 500nM, 700nM, 900nM, 1uM, 2.5uM, 5uM, 7.5uM, 10uM, 12.5uM, 15uM, 17.5uM, 20uM or 22uM) to obtain ectoderm cells ; wherein the NouvNeu-basal base medium comprises Duchenne's modified Eagle/F12 medium (DMEM/F12 medium), 0.1%-2% Minimum Essential Medium Non-Essential Amino Acids (the optimal concentration is 1%), 0.1-0.8 g/L sodium chloride (the optimal concentration is 0.5g/L), 0.1-80mg/L vitamin C (L-ascorbic acid, the optimal concentration is 64mg/L), 0.1-1.8uM vitamin B12 (the optimal concentration is 1.2uM), 0.1-9.8ng/ml progesterone (the optimal concentration is 6.3ng/ml), 10-35ug/ml putrescine (the optimal concentration is 23ug/ml), 1ug /L-25ug/L sodium selenite (the optimal concentration is 13.6ug/L), 0.1-44ug/ml insulin (the optimal concentration is 22ug/ml), 0.5-20umol/L Optiferrin (the optimal concentration is 10µmol/ L), 2.5-100ng/ml plant-derived recombinant human basic growth factor (OsrbFGF, the optimal concentration is 50ng/mL).

In one aspect, the present invention provides use of LY2157299 and JW55 in the differentiation of (for example, human-derived) pluripotent stem cells into neural stem cells.

In another aspect, the present invention provides a medium for differentiating (for example, human-derived) pluripotent stem cells into neural stem cells, wherein LY2157299 and JW55 are added to a NouvNeu-basal base medium; wherein the NouvNeu-basal base medium comprises Duchenne's modified Eagle/F12 medium (DMEM/F12 medium), 0.1%-2% Minimum Essential Medium Non-Essential Amino Acids (the optimal concentration is 1%), 0.1-0.8 g/L sodium chloride (the optimal concentration is 0.5g/L), 0.1-80mg/L vitamin C (L-ascorbic acid, the optimal concentration is 64mg/L), 0.1-1.8uM vitamin B12 (the optimal concentration is 1.2uM), 0.1-9.8ng/ml progesterone (the optimal concentration is 6.3ng/ml), 10-35ug/ml putrescine (the optimal concentration is 23ug/ml), 1ug /L-25ug/L sodium selenite (the optimal concentration is 13.6ug/L), 0.1-44ug/ml insulin (the optimal concentration is 22ug/ml), 0.5-20umol/L Optiferrin (the optimal concentration is 10µmol/ L), 2.5-100ng/ml plant-derived recombinant human basic growth factor (OsrbFGF, the optimal concentration is 50ng/mL); optionally, the concentration of JW55 is 0.5-20uM, and the optimal concentration is SuM; optionally, the amount of LY2157299 is 55nM-25uM, preferably 100nM, 300nM, 500nM, 700nM, 900nM, 1uM, 2.5uM, 5uM, 7.5uM, 10uM, 12.5uM, 15uM, 17.5uM, 20uM or 22uM.

In another aspect, the present invention provides a medium for differentiating (for example, human-derived) ectoderm into neural stem cells, wherein LY2157299 and JW55 are added to a NouvNeu-basal base medium; wherein the NouvNeu-basal base medium comprises Duchenne's modified Eagle/F12 medium (DMEM/F12 medium), 0.1%-2% Minimum Essential Medium Non-Essential Amino Acids (the optimal concentration is 1%), 0.1-0.8 g/L sodium chloride (the optimal concentration is 0.5g/L), 0.1-80mg/L vitamin C (L-ascorbic acid, the optimal concentration is 64mg/L), 0.1-1.8uM vitamin B12 (the optimal concentration is 1.2uM), 0.1-9.8ng/ml progesterone (the optimal concentration is 6.3ng/ml), 10-35ug/ml putrescine (the optimal concentration is 23ug/ml), 1ug /L-25ug/L sodium selenite (the optimal concentration is 13.6ug/L), 0.1-44ug/ml insulin (the optimal concentration is 22ug/ml), 0.5-20umol/L Optiferrin (the optimal concentration is 10µmol/ L), 2.5-100ng/ml plant-derived recombinant human basic growth factor (OsrbFGF, the optimal concentration is 50ng/mL); optionally, the concentration of JW55 is 0.5-20uM JW55, and the optimal concentration is SuM; optionally, the amount of LY2157299 is 55nM-25uM, preferably 100nM, 300nM, 500nM, 700nM, 900nM, 1uM, 2.5uM, 5uM, 7.5uM, 10uM, 12.5uM, 15uM, 17.5uM, 20uM or 22uM.

In another aspect, the present invention provides a method for differentiating (for example, human-derived) pluripotent stem cells into neural stem cells, wherein the pluripotent stem cells are cultured in the above medium for differentiating (for example, human-derived) pluripotent stem cells into neural stem cells.

In another aspect, the present invention provides a method for differentiating (for example, human-derived) ectoderm cells into neural stem cells, wherein the ectoderm cells are cultured in the above medium for differentiating (for example, human-derived) ectoderm cells into neural stem cells.

In one aspect, the present invention provides use of LY2157299 and AM580 in the differentiation of (for example, human-derived) pluripotent stem cells into photoreceptor neuron cells.

Further, the amount of LY2157299 is 55nM-25uM, preferably 100nM, 300nM, 500nM, 700nM, 900nM, 1uM, 2.5uM, 5uM, 7.5uM, 10uM, 12.5uM, 15uM, 17.5uM, 20uM or 22uM.

Further, the amount of AM580 is 0.05-0.95uM, preferably 0.08uM, 0.09uM, O.luM, 0.2uM, 0.3uM, 0.5uM, 0.6uM, 0.7uM, 0.8uM, 0.9uM.

In another aspect, the present invention provides an induction medium for differentiation from (for example, human-derived) ectoderm into photoreceptor neuron cells, wherein LY2157299 and AM580 are added to a NouvNeu-basal base medium; the NouvNeu-basal base medium comprises Duchenne's modified Eagle/F12 medium (DMEM/F12 medium), 0.1%-2% Minimum Essential Medium Non-Essential Amino Acids (the optimal concentration is 1%), 0.1-0.8 g/L sodium chloride (the optimal concentration is 0.5g/L), 0.1-80mg/L vitamin C (L-ascorbic acid, the optimal concentration is 64mg/L), 0.1-1.8uM vitamin B12 (the optimal concentration is 1.2uM), 0.1-9.8ng/ml progesterone (the optimal concentration is 6.3ng/ml), 10-35ug/ml putrescine (the optimal concentration is 23ug/ml), 1ug /L-25ug/L sodium selenite (the optimal concentration is 13.6ug/L), 0.1-44ug/ml insulin (the optimal concentration is 22ug/ml), 0.5-20umol/L Optiferrin (the optimal concentration is 10µmol/ L), 2.5-100ng/ml plant-derived recombinant human basic growth factor (OsrbFGF, the optimal concentration is 50ng/mL).

Further, the amount of the LY2157299 is 55nM-25uM, preferably 100nM, 300nM, 500nM, 700nM, 900nM, 1uM, 2.5uM, 5uM, 7.5uM, 10uM, 12.5uM, 15uM, 17.5uM, 20uM or 22uM.

Further, the amount of the AM580 is 0.05-0.95uM, preferably 0.08uM, 0.09uM, O.luM, 0.2uM, 0.3uM, 0.5uM, 0.6uM, 0.7uM, 0.8uM, 0.9uM.

Further, the amount of the LY2157299 is 7.5uM, and the amount of the AM580 is 0.5uM.

In another aspect, the present invention provides a method for differentiating pluripotent stem cells into photoreceptor neuron cells, wherein the pluripotent stem cells are cultured in a NouvNeu-basal base medium supplemented with 55nM-25uM LY2157299 (preferably 100nM, 300nM, 500nM, 700nM, 900nM, 1uM, 2.5uM, 5uM, 7.5uM, 10uM, 12.5uM, 15uM, 17.5uM, 20uM or 22uM) to obtain ectoderm cells; afterwards, the ectoderm cells are cultured in the above-mentioned photoreceptor neuron cell induction medium to obtain photoreceptor neuron cells; preferably, the culture is performed in the presence of basement membrane; more preferably, the basement membrane is a combination of one or more of Matrigel (STEMCELL Technologies), laminin and vitronectin; wherein the NouvNeu-basal base medium comprises Duchenne's modified Eagle/F12 medium (DMEM/F12 medium), 0.1%-2% Minimum Essential Medium Non-Essential Amino Acids (the optimal concentration is 1%), 0.1-0.8 g/L sodium chloride (the optimal concentration is 0.5g/L), 0.1-80mg/L vitamin C (L-ascorbic acid, the optimal concentration is 64mg/L), 0.1-1.8uM vitamin B12 (the optimal concentration is 1.2uM), 0.1-9.8ng/ml progesterone (the optimal concentration is 6.3ng/ml), 10-35ug/ml putrescine (the optimal concentration is 23ug/ml), 1ug /L-25ug/L sodium selenite (the optimal concentration is 13.6ug/L), 0.1-44ug/ml insulin (the optimal concentration is 22ug/ml), 0.5-20umol/L Optiferrin (the optimal concentration is 10µmol/ L), 2.5-100ng/ml plant-derived recombinant human basic growth factor (OsrbFGF, the optimal concentration is 50ng/mL).

In another aspect, the present invention provides photoreceptor neuron cells, which are obtained by using the above induction medium for differentiating (for example, human-derived) ectoderm into photoreceptor neuron cells.

In another aspect, the present invention provides photoreceptor neuron cells, which are prepared by using the above method of differentiating (for example, human-derived) pluripotent stem cells into photoreceptor neuron cells.

In another aspect, the present invention provides neural stem cells, which are obtained by using the above induction medium for differentiating (for example, human-derived) ectoderm cells into neural stem cells.

In another aspect, the present invention provides neural stem cells, which are obtained by using the above method for differentiating (for example, human-derived) ectoderm cells into neural stem cells.

In another aspect, the invention provides ectoderm cells, which are obtained by using the above medium for differentiating (for example, human-derived) pluripotent stem cells into (for example, human-derived) ectoderm cells.

In another aspect, the invention provides ectoderm cells, which are prepared by the above method for differentiating (for example, human-derived) pluripotent stem cells into (for example, human-derived) ectoderm cells.

In another aspect, the present invention relates to use of the photoreceptor neuron cells in the treatment of degenerative diseases in optic nerve, such as age-related macular degeneration and pigmental degeneration.

In another aspect, the present invention relates to use of the photoreceptor neuron cells in photoreceptor nerve cell transplantation.

In another aspect, the present invention relates to use of the ectoderm cells in the preparation of photoreceptor neuron cells.

The present invention relates to use of compound LY2157299 and/or JW55 in the development of nervous system in vitro, to the combination of the compound and a base medium, as well as to use of the combination in the culture of ectoderm cells or neural stem cells, and to the clinical application of the combination.

The present invention relates to use of a compound (for example, LY2157299 and/or AM580) in the development of nervous system in vitro, to the combination of the compound and a base medium, as well as to use of the combination in the culture of photoreceptor nerve cells, and to the clinical application of the combination.

The present invention relates to a kit comprising a NouvNeu-basal base medium, LY2157299, and/or AM580; preferably, the amount of LY2157299 is 55nM-25uM, preferably 100nM, 300nM, 500nM, 700nM, 900nM, 1uM, 2.5uM, 5uM, 7.5uM, 10uM, 12.5uM, 15uM, 17.5uM, 20uM or 22uM; the amount of AM580 is 0.05-0.95uM, preferably 0.08uM, 0.09uM, O.luM, 0.2uM, 0.3uM, 0.5uM, 0.6uM, 0.7uM, 0.8uM, 0.9uM.

The steps of induction of photoreceptor neuron cells are as follows:
Pluripotent stem cells are subjected to a monolayer adherent culture in a serum-free ectoderm induction medium for 15 days to obtain adherent neural stem cells, wherein the serum-free ectoderm induction medium comprises chemical small molecules, amino acids, inorganic salts and other components used in the present invention, but does not comprise components such as serum, substances acting on BMP or TGF signal transduction pathway. The obtained cells are further treated by a vitamin pathway activator to obtain photoreceptor cells.

In a specific embodiment, the substances acting on BMP signal transduction pathway include one or more the following proteins in any combination: BMP2, BMP4, BMP4, Smad1, Smad5, Smad8; wherein the substances acting on TGF signal transduction pathway include one or more the following proteins in any combination: Activin, TGF-beta, Nodal, Smad2, Smad3. The vitamin includes one or more vitamins in any combination: vitamin C, vitamin E, vitamin B12, vitamin A.

In a specific embodiment, the adherent culture is performed in the presence of a basement membrane preparation. The basement membrane preparation of the present invention can form a thin film composed of extracellular matrix molecules on the surface of culture vessel, and can provide support similar to the *in vivo* environment for parameters such as cell morphology, growth and differentiation, and movement. In a specific embodiment, the basement membrane is a combination of one or more of Matrigel (STEMCELL Technologies), Laminin and Vitronectin.

The serum-free medium in the present invention means that it does not contain serum directly separated from blood. Serum is the clear liquid portion of plasma that does not contain fibrinogen or blood cells and remains liquid after blood clots. The serum-free medium may contain serum replacements. Examples of serum replacements include purified substances such as serum albumin, transferrin, and fatty acids.

In the present invention, the expressions "photoreceptor neuron cells", "photoreceptor nerve cells" and "photoreceptor cells" are interchangeable.

### BRIEF DESCRIPTION OF THE DRAWINGS

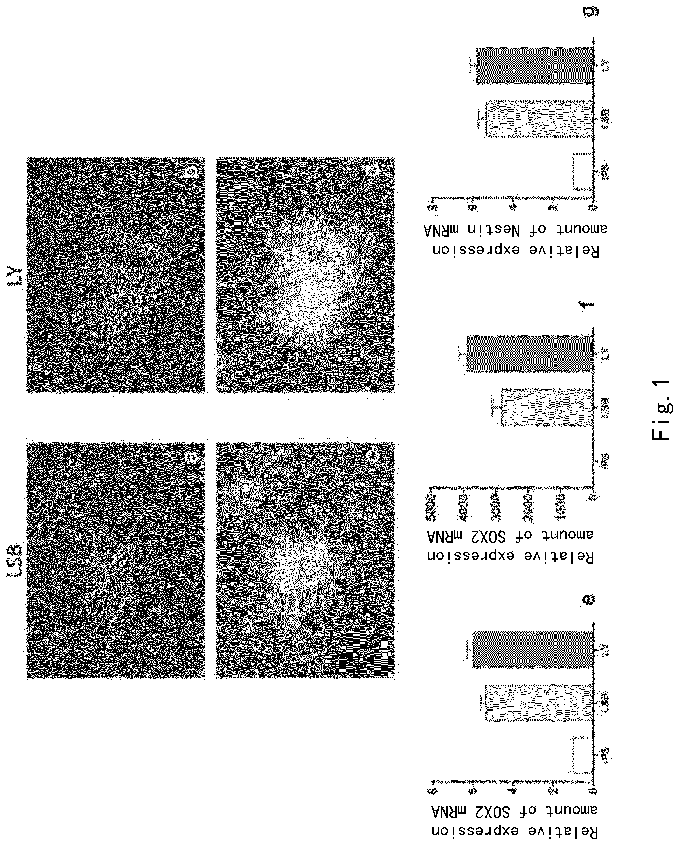

Figure 1 shows the ectoderm cells obtained by using LY2157299, the differentiation of the obtained cells into neural stem cells and the molecular identification results thereof. Figure 1a shows the neural stem cells obtained by using a control method, wherein the structure of the neural rosette is reconstructed after passage in vitro; Figure 1b shows the neural stem cells obtained by using LY2157299, wherein the structure of the neural rosette is reconstructed after passage in vitro; Figure 1c shows the neural stem cells obtained by using a control method, which can express neural stem cell markers Pax6 and Nestin after in vitro reconstruction of neural rosettes; Figure 1d shows neural stem cells obtained by using LY2157299, which can express neural stem cell markers Pax6 and Nestin after in vitro reconstruction of neural rosettes; Figures 1e-1f show the difference in the expression of neural stem cell markers during the formation of neural stem cells by using Q-PCR between the method using small molecule inhibitors of the present invention and the control induction method (CK). The above results show that LY2157299 can not only form neural stem cells, but also slightly increase the expression of neural stem cell markers compared with the control method.

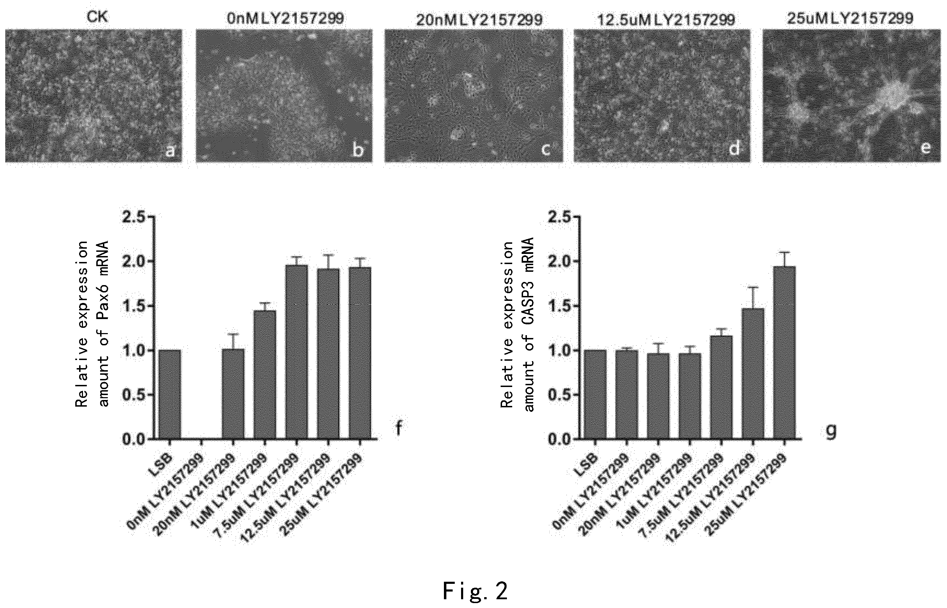

Figure 2 shows the induction of ectoderm cells by using different concentrations of LY2157299, and the screening of optimal concentration. Figure 2a shows the morphology of ectoderm cells formed by using LSB control method; Figure 2b shows that in the absence of LY2157299, cells do not form ectodermal morphology; Figure 2c shows that low concentration of LY2157299 achieves differentiation into mixed cell including ectodermal cells; Figure 2d shows that an appropriate concentration of LY2157299 produces uniformly differentiated ectoderm cells; Figure 2e shows that excessively high concentration of LY2157299 promote the differentiation of ectoderm cells into neural cells; Figure 2f shows that ectoderm markers are positively correlated with the concentration of LY2157299; Figure 2g shows that apoptotic markers are positively correlated with the concentration of LY2157299. Therefore, the optimal concentration of LY2157299 used in the present invention is 7.5uM.

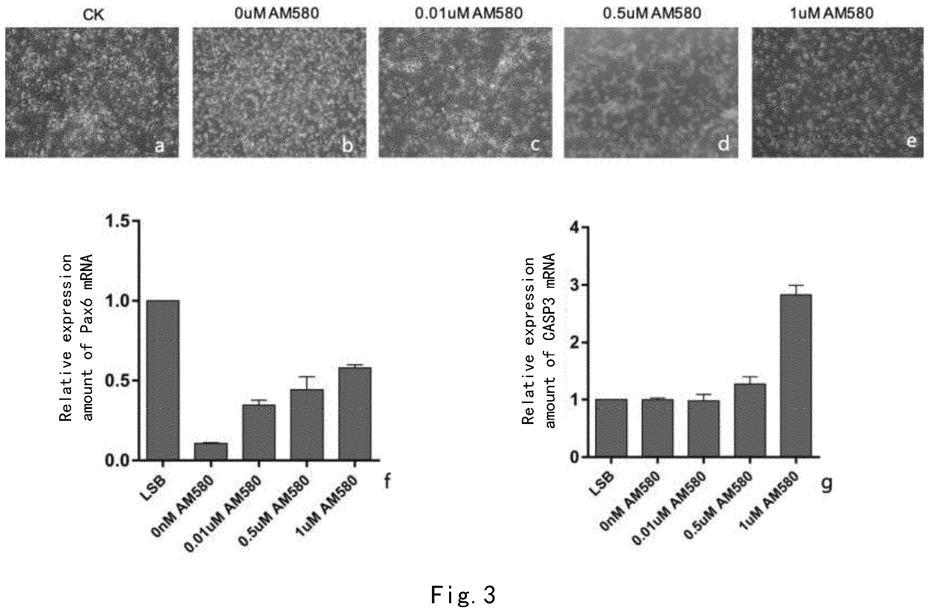

Figure 3 shows the induction of photoreceptor cells by using different concentrations of AM580, and the screening of optimal concentration. Figure 3a shows the morphology of ectoderm cells formed by using LSB control method; Figure 3b shows that in the absence of AM580, cortical neuron morphology with long axons is formed; Figure 3c shows that low concentration of AM580 achieves differentiation into mixed cells including cortical neurons; Figure 3d shows that an appropriate concentration of AM580 achieves uniformly differentiated photoreceptor cells with short axons; Figure 3e shows that excessively high concentration of AM580 results in stress in differentiated cells; Figure 3f shows that ectoderm and photoreceptor markers PAX6 are positively correlated with the concentration of AM580; Figure 3g shows that apoptotic markers are positively correlated with the concentration of AM580. Therefore, the optimal concentration of AM580 in the present invention is 0.5uM.

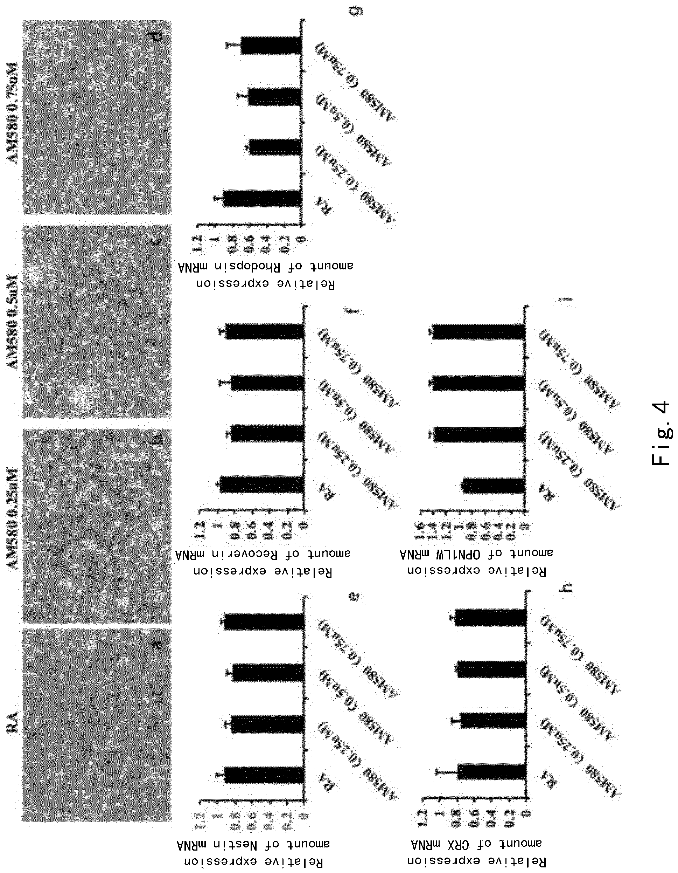

Figure 4 shows the induction of photoreceptor cells by using different concentrations of AM580, the morphology of the obtained cells and the Q-PCR identification of photoreceptor markers. Figure 4a-4d shows the photoreceptor cells obtained by RA control and different concentrations of AM580; Figure 4e shows the comparison of expression of neural cell marker Nestin in the cells used in 4a-4d; Figure 4f shows the comparison of expression of photoreceptor cell marker Rcoverin in the cells used in 4a-4d; Figure 4g shows the comparison of expression of photoreceptor cell marker Rhodopsin in the cells used in 4a-4d; Figure 4h shows the comparison of expression of photoreceptor cell marker CRX in the cells used in 4a-4d; Figure 4i shows the comparison of expression of photoreceptor cell marker Opsin1 (OPN1LW) in the cells used in 4a-4d.

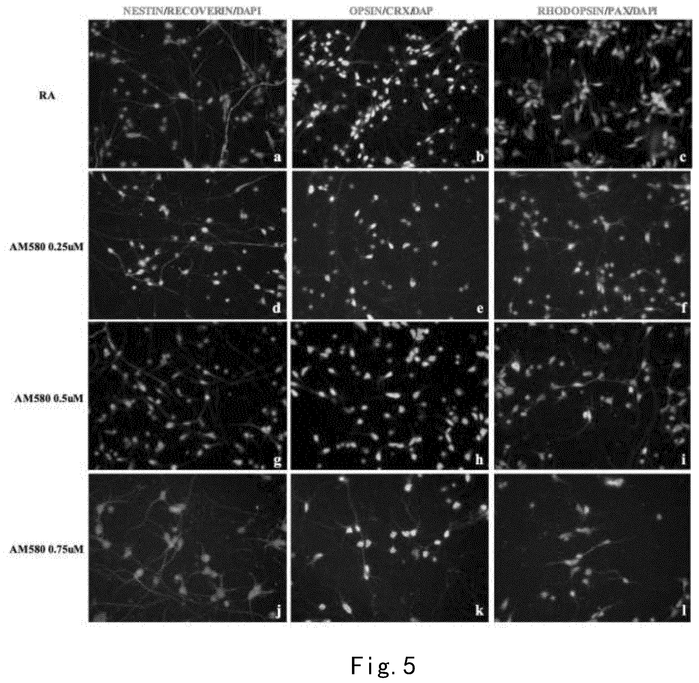

Figure 5 shows the induction of photoreceptor cells by using different concentrations of AM580, the morphology of the obtained cells and the fluorescent immunoassay of photoreceptor markers. The results shows that the photoreceptor cells obtained in RA control group express Nestin protein and Recoverin protein (Sa), mature photoreceptor marker proteins CRX and Opsin protein (5b), Rhodopsin and PAX6 protein (5c); the photoreceptor cells obtained in groups with different concentrations of AM580 also have the same protein expression patterns with that in the RA control group (5d-5l).

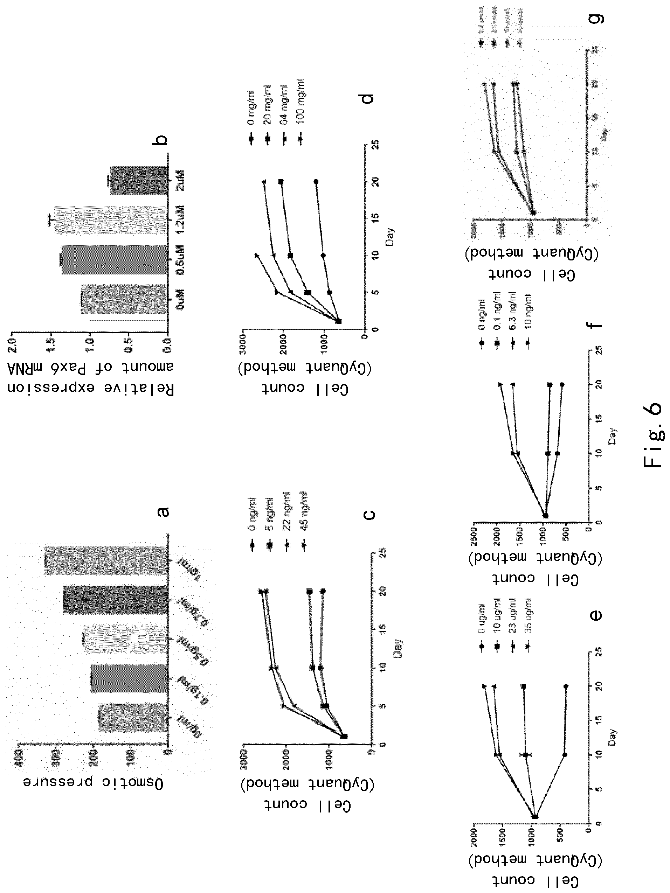

Figure 6 is a schematic diagram showing the concentration identification of key components in the culture system. 6A shows the influence of different concentrations of sodium chloride on the osmotic pressure of the culture system; 6b shows the expression difference of photoreceptor neuron cells obtained by using different concentrations of vitamin B12; 6c shows the influence of insulin on the physiological state of cells during long-term culture; 6d shows the influence of vitamin C on the physiological state of cells during long-term culture of cells; 6e shows the influence of different concentrations of progesterone on the physiological state of cells during the induction of photoreceptor cells; 6f shows the influence of different concentrations of putrescine on the physiological state of cells during the induction of photoreceptor cells; 6g shows the influence of different concentrations of Optiferrin on the physiological state of cells during the induction of photoreceptor cells.

### Advantages

The invention establishes an induction culture system of photoreceptor neuron which is serum-free and animal-derived substance-free. Therefore, photoreceptor neurons are obtained by reprogramming and directional differentiation of somatic cells from multiple sources (such as human-derived), and they have various complete biological functions with stable states and high purities among multiple batches, which solve the problems of low purity and long cycle in the production process of cell-based drugs. The invention also solves the long-standing problems involving animal-derived culture methods and trophoblastic cell contamination, and can be used for transplantation therapy for various visual impairment diseases, thus has huge economic and social effects.

### DETAILED DESCRIPTION OF THE INVENTION

The implementation process and beneficial effects of the present invention are described in detail below through specific examples, which are intended to help readers better understand the essence and characteristics of the present invention, and are not intended to limit the scope of the present application.

### Example 1. Preparation of Ectoderm Cell Induction Medium

The formula of the induction medium (hereinafter referred to as NouvNeu-basal):
Duchenne's modified Eagle/F12 medium (DMEM/F12 medium, SIGMA), 0.1%-2% Minimum Essential Medium Non-Essential Amino Acids (cat No. 11140076, Thermo Fisher), 0.1-0.8 g/L sodium chloride (0.5g/L, SIGMA), 0.1-80mg/L vitamin C (L-ascorbic acid, 64mg/L, SIGMA), 0.1-1.8uM vitamin B12 (1.2uM, SIGMA), 0.1-9.8ng/ml progesterone (6.3ng/ml, SIGMA), 10-35ug/ml putrescine (23ug/ml, SIGMA), 1ug /L-25ug/L sodium selenite (the optimal concentration is 13.6ug/L, SIGMA), 0.1-44ug/ml insulin (22ug/ml, Solarbio), 0.5-20umol/L Optiferrin (10µmol/L, SIGMA), 2.5-100ng/ml plant-derived recombinant human basic growth factor (OsrbFGF, the optimal concentration is 50ng/mL, Healthgen Biotech).

The ectoderm cell induction medium of the present invention was obtained by supplementing the NouvNeu-basal base medium with 55nM-25uM LY2157299 (Selleck) (with an optimal concentration of 7.5uM) (preferably 100nM, 300nM, 500nM, 700nM, 900nM, 1uM, 2.5uM, 5uM, 7.5uM, 10uM, 12.5uM, 15uM, 17.5uM, 20uM or 22uM). An induction method using said ectoderm cell induction medium was hereinafter referred to as a LY induction method.

In a control experiment, 100 nM LDN-193189 (Selleck) and 10 uM SB431542 (Selleck) were added to the NouvNeu basal base medium, hereinafter referred to as a LSB induction method.

### Example 2. Induction and identification of ectoderm

### 2.1 Chemical induction of ectoderm

Ectoderm cells were derived from human pluripotent stem cells, including embryogenic pluripotent stem cells, such as H9 cell line and human induced pluripotent stem cells. Human induced pluripotent stem cells were obtained by reprogramming from CD34+ cells according to "Reprogrammed culture medium and culture method of reprogrammed induced multipotential stem cell" (ZL 201910050800.7). In the present invention, "induced pluripotent stem cells" and "pluripotent stem cells" were used interchangeably.

For human pluripotent stem cells, a T25 cell culture flask was coated with Matrigel (STEMCELL Technologies), and incubated in an incubator at37°C for more than one hour. 1×10⁶ cells were inoculated in a T25 culture flask for expansion and passage.

During the induction of ectoderm cells, a 6-well culture plate was coated by using 50ug/ml polylysine (SIGMA, P6407), and incubated in an incubator at 37°C for more than 3 hours; then further coated by 5ug/ml laminin (Laminin, I2020, SIGMA ALDRICH), and incubated in an incubator at 37°C for more than 3 hours after plating. After the pluripotent stem cells reached 70% coverage, they were digested with EDTA at 37 °C for 5 min, and the cell digestion was terminated with DMEM. After washing and centrifugation, the cells were re-plated in the T25 culture plate at 2×10⁵ cells per flask. The neural stem cell induction medium of the present invention was used for induction, and replaced with fresh induction medium every day until lamellar ectoderm cells were formed.

Ectoderm was digested with EDTA for 5 minutes at 37°C, and the cell digestion was terminated with DMEM. After washing and centrifuging, the cells were re-seeded in a T25 culture plate coated with 50ug/ml polylysine and 5ug/ml laminin at 2×10⁵ cells per flask for cell reproduction culture. The condition for culture was at 37°C, 5% CO₂ (Panasonic, MCO-18AC).

### 2.2 Identification of ectoderm cells

Ectoderm cells could be further cultured to form neural stem cells. In order to identify this ability of ectoderm cells, the ectoderm cells obtained in Example 2.1 were further cultured *in vitro* by using a NouvNeu-basal base medium supplemented with 7.5uM LY2157299 and 0.5-20uM JW55 (with an optimal concentration of 5uM) until a neural rosette-like structure was formed. The LSB induction method was used as a control.

The neural stem cells differentiated from the ectoderm obtained by the LY induction method and the LSB induction method were respectively taken and identified by immunofluorescence staining as follows: cells were fixed with 4% paraformaldehyde at room temperature for 40 minutes, washed twice with DPBS buffer; then permeabilized with 0.1% Triton X-100 for 5 minutes, washed twice with DPBS buffer; then incubated overnight at 4°C with DPBS buffer containing 10% horse serum and 0.1% Triton X-100; added with antibodies diluted in DPBS buffer, incubated at 37°C for 2 hours, washed three times with DPBS buffer, and then photographed by Leica DMi8. The details for the antibodies were shown in Table 1. The results were shown in Figures 1a-1d, showing that neural stem cells with a neural rosette structure could be produced by both the LY induction method and the control LSB induction method, and the obtained neural stem cells could express Pax6 and Nestin.

**Table 1. Antibodies used for immunofluorescence staining of cells**

| Primary antibody | Concentration of primary antibody | Secondary antibody | Concentration of secondary antibody |
|---|---|---|---|
| Anti-PAX6-antibody < ThermoFisher) | 1:200 | Donkey anti-mouse IgG(H+L), Alexa Fluor 568 (Invitrogen A11008) | 1:1000 |
| Anti-Nestin antibody (CST) | 1:400 | Goat anti-rabbit IgG(H+L), Alexa Fluor 488 (Invitrogen A11008) | 1:1000 |

Transcription changes of different marker genes during induction from ectoderm cells to neural stem cells were detected by Q-PCR. The neural stem cells obtained from the ectoderm cells in Example 2 by using the LY induction method and the LSB induction control method were respectively extracted with RNeasy Mini or Micro Kit (QIAGEN) for total RNA, and cDNA was synthesized by 1 mg RNA via SuperScript III First-Strand Synthesis System (Invitrogen). SYBR Premix Ex Taq (TaKaRa) and Thermal Cycler Dice Real Time System (TaKaRa) were used for labeling and reaction by Quantitative PCR, and beta-Actin was used as internal control. All data were analyzed by delta-Ct method. Triplicates were used for each group of experiments, and ANOVA was performed. Primer sequences used to identify coding genes of different cellular markers were shown in Table 2. The results were shown in Figures 1e-1g. The results showed that the neural stem cells obtained by the LY induction method and the control LSB induction method expressed various neural stem cell markers SOX2, PAX6 and Nestin at the transcriptional level. Therefore, the ectoderm obtained in the present invention possessed an ability to further differentiate into neural stem cells.

### 2.3 The effect of different concentrations of LY2157299 on the transcription level of ectoderm cells

The effect of different concentrations of LY2157299 on the formation of ectoderm cells was detected by Q-PCR. The ectoderm cells obtained from the induced pluripotent stem cells in Example 2 by using different concentrations of LY2157299 in the LY induction method and LSB induction method were respectively extracted with RNeasy Mini or Micro Kit (QIAGEN) for total RNA, and cDNA was synthesized by 1 mg RNA via SuperScript III First-Strand Synthesis System (Invitrogen). SYBR Premix Ex Taq (TaKaRa) and Thermal Cycler Dice Real Time System (TaKaRa) were used for labeling and reaction by Quantitative PCR, and beta-Actin was used as internal control. All data were analyzed by delta-Ct method. Triplicates were used for each group of experiments, and ANOVA was performed. Primer sequences used to identify coding genes of different cellular markers were shown in Table 2. As shown in Figure 2, the results show that in the absence of LY2157299 or at low concentration of LY2157299, the ectoderm could not be completely formed, and high concentration of LY2157299 induced cell apoptosis, so the concentration used in the present invention was within the optimal concentration range of LY2157299.

**Table 2. Primers for detection of ectoderm and neural stem cells**

| | |
|---|---|
| SOX2-F | CATGCAGGTTGACACCGTTGG (SEQ ID NO:1) |
| SOX2-R | ATGGATTCTCGGCAGACTGATTCA (SEQ ID NO:2) |
| PAX6-F | TCTTTGCTTGGGAAATCCG (SEQ ID NO:3) |
| PAX6-R | CTGCCCGTTCAACATCCTTAG (SEQ ID NO:4) |
| Nestin-F | TCAAGATGTCCCTCAGCCTGGA (SEQ ID NO:9) |
| Nestin-R | TGGCACAGGTGTCTCAAGGGTAG (SEQ ID NO:10) |
| CASP3-F | GGAAGCGAATCAATGGACTCTGG (SEQ ID NO:5) |
| CASP3-R | GCATCGACATCTGTACCAGACC (SEQ ID NO:6) |
| ACTIN-F | TCCCTGGAGAAGAGCTACGA (SEQ ID NO:7) |
| ACTIN-R | AGCACTGTGTTGGCGTACAG (SEQ ID NO:8) |

### Example 3. Differentiation of ectoderm cells into photoreceptor neuron cells

The ectoderm cells obtained by the NouvNeu system in Example 2 were subjected to directed differentiation in NouvNeu medium. For differentiation, a 6-well culture plate was coated by 50ug/ml poly-lysine (SIGMA, P6407), incubated in an incubator at 37°C for more than 3 hours until cells were seeded.

The photoreceptor neuron cell induction medium of the present invention was obtained by supplementing the NouvNeu-basal base medium with 55nM-25uM LY2157299 (with an optimal concentration of 7.5uM) (preferably 100nM, 300nM, 500nM, 700nM, 900nM, 1uM, 2.5uM, 5uM, 7.5uM, 10uM, 12.5uM, 15uM, 17.5uM, 20uM or 22uM) and 0.05-0.95uM AM580 (Selleck) (with an optimal concentration of 0.5uM) (preferably 0.08uM, 0.09uM, 0.1uM, 0.2uM, 0.3uM, 0.5uM, 0.6uM, 0.7uM, 0.8uM, 0.9uM). The cells were cultured until photoreceptor neuron cell were formed. A control experiment was performed by adding 0.5uM retinoic acid to the NouvNeu medium, hereinafter referred to as RA.

The screening test of AM580 concentration was carried out with reference to Example 2.3, and the expression difference of photoreceptor neuron cells obtained by using different concentrations of AM580 in the AM580 induction method and the LSB induction method was compared. See Table 2 and Table 4 for the details of primers used. As shown in Figure 3, the results showed that the concentration of AM580 was positively correlated with the photoreceptor non-specific marker PAX6, but when the concentration of AM580 was too high, it would lead to apoptosis. In combination with the effect of AM580 on the expression of photoreceptor neuron-specific genes shown in Figure 4, it could be concluded that the concentrations of AM580 used in the present invention are within the range of optimal concentration.

The medium was replaced with fresh medium every 3 days until day 21. The conditions for culture were at 37°C, 5% CO₂ (Panasonic, MCO-18AC). After the induction was completed, the samples in experimental group and control group were respectively identified by immunofluorescence staining as follows: cells were fixed with 4% paraformaldehyde at room temperature for 40 minutes, washed twice with DPBS buffer; then permeabilized with 0.1% Triton X-100 for 5 minutes, washed twice with DPBS buffer; then incubated overnight at 4°C with DPBS buffer containing 10% horse serum and 0.1% Triton X-100; added with a primary antibody diluted in DPBS buffer containing 2% horse serum and 0.1% Triton X-100, incubated at 37 °C for two hours, and washed three times with DPBS buffer, added with a secondary antibody diluted in DPBS buffer containing 2% horse serum and 0.1% Triton X-100, and then photographed. The details for the antibodies were shown in Table 3. The experimental results showed that the photoreceptor neurons obtained in Example 3 were identical to those obtained in RA control, which had axonal structures , and expressed the photoreceptor neuron markers OPSIN, CRX, RECOVERIN, RHODOPSIN and PAX6, as well as the neural lineage marker Nestin (Figure 5) .

**Table 3. Antibodies used for directed differentiation immunofluorescence staining**

| Primary antibody | Concentration of primary antibody | secondary antibody | Concentration of secondary antibody |
|---|---|---|---|
| Anti-PAX6-antibody < Abcam) | 1:200 | Goat anti-rabbit IgG(H+L), Alexa Fluor 488 (Invitrogen A10037) | 1:1000 |
| Anti-NESTIN antibody (MAB5326) | 1:200 | Donkey anti-mouse IgG(H+L), Alexa Fluor 568 (Invitrogen A11008) | 1:1000 |
| Anti-Recoverin antibody (AB5585) | 1:50 | Goat anti-rabbit IgG(H+L), Alexa Fluor 488 (Invitrogen A10037) | 1:1000 |
| Anti-Opsin antibody (AB5405) | 1:1000 | Goat anti-rabbit IgG(H+L), Alexa Fluor 568 (Abcam175713) | 1:1000 |
| Anti-Rhodopsin antibody (MAB5316) | 1:200 | Goat Anti-Rat IgG(H+L) , Alexa Fluor 568 ( Abcam175476) | 1:1000 |
| Anti-CRX antibody (4G11) | 1:100 | Donkey anti-mouse IgG(H+L),Alexa Fluor 488 (Abcam181289) | 1:1000 |

Transcription changes of different marker genes during induction from ectoderm cells to photoreceptor neuron cells were detected by Q-PCR. The photoreceptor cells obtained by using the present invention and different concentrations of AM580, and the photoreceptor cells obtained by the RA control induction method were respectively extracted with RNeasy Mini or Micro Kit (QIAGEN) for total RNA, and cDNA was synthesized by 1 mg RNA via SuperScript III First-Strand Synthesis System (Invitrogen). SYBR Premix Ex Taq (TaKaRa) and Thermal Cycler Dice Real Time System (TaKaRa) were used for labeling and reaction of Quantitative PCR, and beta-Actin was used as internal control. All data were analyzed by delta-Ct method. Three replicates were used for each group of experiments, and ANOVA was performed. Primer sequences used to identify coding genes of different cellular markers were shown in Table 4. The results showed that the AM580 could induce the expression of multiple photoreceptor cell-specific marker genes, which was not significantly lower than that of the RA control (Figure 4).

**Table 4. Primers for different marker gene during photoreceptor cell induction**

| | |
|---|---|
| CRX-F | CTTGTAGAGGACGCAGTCTCCATCT (SEQ ID NO:11) |
| CRX-R | CAATCGTGCCAGACGGTGTTAGG (SEQ ID NO:12) |
| OPN1LW-F | TCATCGCCAGCACTATCAGCATTG (SEQ ID NO:13) |
| OPN1LW-R | ACAGCAGACCAGATCCAGGAGAAG (SEQ ID NO:14) |
| Recoverin-F | CAGTTCCAGAGCATCTACGCCAAG (SEQ ID NO:15) |
| Recoverin-R | GCCATGACGATCTCCAGCACTTC (SEQ ID NO:16) |
| Rhodopsin-F | CAGCGTGGCATTCTACATCTTCAC (SEQ ID NO:17) |
| Rhodopsin-R | ACCTGGCTCGTCTCCGTCTT (SEQ ID NO:18) |
| PAX6-F | TCTTTGCTTGGGAAATCCG (SEQ ID NO:19) |
| PAX6-R | CTGCCCGTTCAACATCCTTAG (SEQ ID NO:20) |
| βActin-F | GGCCGAGGACTTTGATTGCACA (SEQ ID NO:21) |
| βActin-R | GGGCACGAAGGCTCATCATTCAA (SEQ ID NO:22) |

### Example 4. Concentration identification of key components in the basal culture system:

The NouvNeu-basal base medium shown in the Examples of the present invention is formulated as: Duchenne's modified Eagle/F12 medium (DMEM/F12 medium), 0.1%-2% Minimum Essential Medium Non-Essential Amino Acids (the optimal concentration is 1%), 0.1-0.8 g/L sodium chloride (the optimal concentration is 0.5g/L), 0.1-80mg/L vitamin C (L-ascorbic acid, the optimal concentration is 64mg/L), 0.1-1.8uM vitamin B12 (the optimal concentration is 1.2uM), 0.1-9.8ng/ml progesterone (the optimal concentration is 6.3ng/ml), 10-35ug/ml putrescine (the optimal concentration is 23ug/ml), 1ug /L-25ug/L sodium selenite (the optimal concentration is 13.6ug/L), 0.1-44ug/ml insulin (the optimal concentration is 22ug/ml), 0.5-20umol/L Optiferrin (the optimal concentration is 10µmol/ L), 2.5-100ng/ml plant-derived recombinant human basic growth factor (OsrbFGF, the optimal concentration is 50ng/mL).

A screening culture system was prepared by using different concentration gradients of sodium chloride while maintaining the optimal concentration of other components. The effect of different concentrations of sodium chloride on the osmotic pressure of culture system was detected by using an automatic freezing point osmometer (FM-8P, Shanghai Medical University Instrument Co., Ltd.), and specific operations were performed according to the product manual (FM-8P, Shanghai Medical University Instrument Co., Ltd.). The test results were shown in Figure 6a in detail, showing that the concentration of sodium chloride increased linearly with the osmotic pressure of the culture system. However, when the concentration reached 1g/ml,, the osmotic pressure of the culture system exceeded the ultimate osmotic pressure 320 mOsm/kg for cell culture, indicating that the concentration of sodium chloride used in the present invention was the most suitable concentration for cell culture.

The screening test of vitamin B12 concentration was carried out with reference to Example 2.3, and the expression difference of photoreceptor neuron cells obtained by using different concentrations of vitamin B12 was compared. See Table 2 and Table 4 for the details of primers used. As shown in Figure 6b, the results showed that the concentration of vitamin B12 was positively correlated with the photoreceptor non-specific marker PAX6, but when the concentration of vitamin B12 was too high, it would lead to non-specific differentiation of cells, resulting in a decrease in the relative expression of PAX6. Therefore, the concentration of vitamin B12 used in the present invention was the optimal concentration.

A screening culture system was prepared by using different concentration gradients of insulin or vitamin C while maintaining the optimal concentration of other components. Ectoderm induction was performed according to the method in Example 2.1. The cell viability was quantitatively detected by Cyquant assay to study the effects of insulin or vitamin C on the physiological state of cells during long-term cell culture. A 96-well opaque cell culture plate was coated, and then the cells are inoculated at 5×10⁴ cells per well, wherein parallel triplicates were set up (the average of the three groups was calculated as the data). The steps in Example 2.1 were repeated with the culture conditions of 37°C, 5% CO₂. Samples were taken at day 1, day 5, day 10 and day 20, respectively, and the cell viability detection was carried out by CyQuant Kit (Invitrogen, X12223) according to the instruction manual, and data were read by SpectraMax i3 Multi-Mode Microplate Reader (VWR, ID3-STD). The results were shown in Figures 6c-6d. Cyquant test showed that the number of cells induced was positively correlated with insulin or vitamin C; however, the number of cells at high concentration of insulin or vitamin C was not significantly different from that obtained by optimal concentration of the present invention. In addition, the high concentration of vitamin C caused the cells to shed in pieces in the late stage of induction, and the induction could not be continued. Therefore, in combination with cost factors, the insulin or vitamin C in the basal medium of the present invention was the optimal concentration.

The Cyquant test was also used to detect the effect of progesterone, putrescine and Optiferrin on cell viability, so as to study the effects of different concentrations of progesterone, putrescine and Optiferrin on the physiological state of cells during the induction of photoreceptor cells. The results were shown in Figure 6e, 6f, and Figure 6g, showing that the concentrations of progesterone, putrescine and Optiferrin were all positively correlated with the cell proliferation rate; combined with cost factors, the ranges of progesterone, putrescine and Optiferrin used in the present invention were the optimal ranges.

It should be noted that the above examples are only preferred specific embodiments of the present invention, and do not limit the present invention in any form. All technical solutions obtained by equivalent replacement or change based on the technical solution of the present invention are within the protection scope of the present invention.

## Claims

1. Use of LY2157299 in the differentiation of pluripotent stem cells into ectoderm, preferably, the amount of the LY2157299 is 55nM-25uM, more preferably 100nM, 300nM, 500nM, 700nM, 900nM, 1uM, 2.5uM, 5uM, 7.5uM, 10uM, 12.5uM, 15uM, 17.5uM, 20uM or 22uM.

2. A culture medium, **characterized in that** it is prepared by adding LY2157299 to a NouvNeu-basal base medium; formula of the NouvNeu-basal base medium is Duchenne's modified Eagle/F12 medium (DMEM/F12 medium), 0.1%-2% Minimum Essential Medium Non-Essential Amino Acids (the optimal concentration is 1%), 0.1-0.8 g/L sodium chloride (the optimal concentration is 0.5g/L), 0.1-80mg/L vitamin C (L-ascorbic acid, the optimal concentration is 64mg/L), 0.1-1.8uM vitamin B12 (the optimal concentration is 1.2uM), 0.1-9.8ng/ml progesterone (the optimal concentration is 6.3ng/ml), 10-35ug/ml putrescine (the optimal concentration is 23ug/ml), 1ug /L-25ug/L sodium selenite (the optimal concentration is 13.6ug/L), 0.1-44ug/ml insulin (the optimal concentration is 22ug/ml), 0.5-20umol/L Optiferrin (the optimal concentration is 10µmol/ L), 2.5-100ng/ml plant-derived recombinant human basic growth factor (OsrbFGF, the optimal concentration is 50ng/mL).

3. The culture medium of claim 2, **characterized in that** the amount of the LY2157299 is 55nM-25uM, preferably 100nM, 300nM, 500nM, 700nM, 900nM, 1uM, 2.5uM, 5uM, 7.5uM, 10uM, 12.5uM, 15uM, 17.5uM, 20uM or 22uM.

4. The culture medium of claim 3, **characterized in that** the amount of the LY2157299 is 7.5uM.

5. A method of differentiating pluripotent stem cells (preferably human-derived pluripotent stem cells) into ectoderm, **characterized in that** the pluripotent stem cells are cultured in the culture medium of any one of claims 2-4 to obtain ectoderm cells.

6. A culture medium, **characterized in that** it is prepared by adding LY2157299 and JW55 to a NouvNeu-basal base medium; wherein the NouvNeu-basal base medium comprises Duchenne's modified Eagle/F12 medium (DMEM/F12 medium), 0.1%-2% Minimum Essential Medium Non-Essential Amino Acids (the optimal concentration is 1%), 0.1-0.8 g/L sodium chloride (the optimal concentration is 0.5g/L), 0.1-80mg/L vitamin C (L-ascorbic acid, the optimal concentration is 64mg/L), 0.1-1.8uM vitamin B12 (the optimal concentration is 1.2uM), 0.1-9.8ng/ml progesterone (the optimal concentration is 6.3ng/ml), 10-35ug/ml putrescine (the optimal concentration is 23ug/ml), 1ug /L-25ug/L sodium selenite (the optimal concentration is 13.6ug/L), 0.1-44ug/ml insulin (the optimal concentration is 22ug/ml), 0.5-20umol/L Optiferrin (the optimal concentration is 10µmol/ L), 2.5-100ng/ml plant-derived recombinant human basic growth factor (OsrbFGF, the optimal concentration is 50ng/mL); optionally, the concentration of JW55 is 0.5-20uM, and the optimal concentration is SuM; optionally, the amount of LY2157299 is 55nM-25uM, preferably 100nM, 300nM, 500nM, 700nM, 900nM, 1uM, 2.5uM, 5uM, 7.5uM, 10uM, 12.5uM, 15uM, 17.5uM, 20uM or 22uM.

7. A method of differentiating pluripotent stem cells (preferably human-derived pluripotent stem cells) into neural stem cells, wherein the pluripotent stem cells are cultured in the culture medium of claim 6.

8. Use of AM580 in the differentiation of pluripotent stem cells (preferably human-derived pluripotent stem cells) into photoreceptor neuron cells, preferably, the amount of AM580 is 0.05-0.95µM, more preferably 0.08uM, 0.09uM, 0.1 uM, 0.2uM, 0.3uM, 0.5uM, 0.6uM, 0.7uM, 0.8uM, 0.9uM.

9. Use of AM580 in the differentiation of ectoderm cells into photoreceptor neuron cells, preferably, the amount of AM580 is 0.05-0.95µM, more preferably 0.08uM, 0.09uM, 0.1 uM, 0.2uM, 0.3uM, 0.5uM, 0.6uM, 0.7uM, 0.8uM, 0.9uM.

10. Use of LY2157299 and AM580 in the differentiation of pluripotent stem cells (preferably human-derived pluripotent stem cells) into photoreceptor neuron cells, preferably, the amount of AM580 is 0.05-0.95µM, preferably 0.08uM, 0.09uM, 0.1 uM, 0.2uM, 0.3uM, 0.5uM, 0.6uM, 0.7uM, 0.8uM, 0.9uM.

11. The use of claim 10, wherein the amount of the LY2157299 is 55nM-25uM, preferably 100nM, 300nM, 500nM, 700nM, 900nM, 1uM, 2.5uM, 5uM, 7.5uM, 10uM, 12.5uM, 15uM, 17.5uM, 20uM or 22uM.

12. A photoreceptor neuron cell induction medium, **characterized in that** it is prepared by adding 55nM-25uM LY2157299 (preferably 100nM, 300nM, 500nM, 700nM, 900nM, 1uM, 2.5uM, 5uM, 7.5 uM, 10uM, 12.5uM, 15uM, 17.5uM, 20uM or 22uM) and 0.05-0.95uM AM580 (preferably 0.08uM, 0.09uM, O.luM, 0.2uM, 0.3uM, 0.5uM, 0.6uM, 0.7uM, 0.8 uM, 0.9uM) to a NouvNeu-basal base medium; wherein the formula of the NouvNeu-basal base medium is Duchenne's modified Eagle/F12 medium (DMEM/F12 medium), 0.1%-2% Minimum Essential Medium Non-Essential Amino Acids (the optimal concentration is 1%), 0.1-0.8 g/L sodium chloride (the optimal concentration is 0.5g/L), 0.1-80mg/L vitamin C (L-ascorbic acid, the optimal concentration is 64mg/L), 0.1-1.8uM vitamin B12 (the optimal concentration is 1.2uM), 0.1-9.8ng/ml progesterone (the optimal concentration is 6.3ng/ml), 10-35ug/ml putrescine (the optimal concentration is 23ug/ml), 1ug /L-25ug/L sodium selenite (the optimal concentration is 13.6ug/L), 0.1-44ug/ml insulin (the optimal concentration is 22ug/ml), 0.5-20umol/L Optiferrin (the optimal concentration is 10µmol/ L), 2.5-100ng/ml plant-derived recombinant human basic growth factor (OsrbFGF, the optimal concentration is 50ng/mL).

13. The photoreceptor neuron cell induction medium of claim 12, **characterized in that** the amount of LY2157299 is 7.5uM, and the amount of AM580 is 0.5uM.

14. A method of differentiating pluripotent stem cells (preferably human-derived pluripotent stem cells) into photoreceptor neuron cells, **characterized in that** the pluripotent stem cells are cultured in the culture medium according to any one of claims 2-4 to obtain ectoderm cells, and then the ectoderm cells are cultured in the photoreceptor neuron cell induction medium according to any one of claims 10-11 to obtain photoreceptor neuron cells, preferably, the culture is performed in the presence of a basement membrane, more preferably, the basement membrane is a combination of one or more of Matrigel, Laminin and Vitronectin.

15. The method of differentiating pluripotent stem cells into photoreceptor neuron cells according to claim 14, **characterized in that** the amount of LY2157299 is 7.5uM.

16. Ectoderm cells, which are cultured by the culture medium according to any one of claims 2-4 or prepared by the method according to claim 5.

17. Photoreceptor neuron cells, which are cultured by the induction medium according to any one of claims 12-13 or prepared by the method according to claim 14 or prepared by the ectoderm cells according to claim 14.

18. Use of the photoreceptor neuron cells according to claim 17 in the treatment of degenerative diseases in optic nerve, such as age-related macular degeneration and pigmental degeneration.

19. Use of the photoreceptor neuron cells according to claim 17 in the photoreceptor nerve cell transplantation.

20. A kit comprising a NouvNeu-basal base medium, LY2157299, and/or AM580; preferably, the amount of LY2157299 is 55nM-25uM, preferably 100nM, 300nM, 500nM, 700nM, 900nM, 1uM, 2.5uM, 5uM, 7.5uM, 10uM, 12.5uM, 15uM, 17.5uM, 20uM or 22uM; the amount of AM580 is 0.05-0.95uM, preferably 0.08uM, 0.09uM, O.luM, 0.2uM, 0.3uM, 0.5uM, 0.6uM, 0.7uM, 0.8uM, 0.9uM.
